# EUROPEAN PATENT APPLICATION

(11) **EP 1 491 192 A1**
(43) Date of publication of application: **29.12.2004**
(21) Application number: 03708639.4
(22) Date of filing: 17.03.2003
(51) Int. Cl.: A61K 31/366, A61K 35/78, A61P 3/06, A61P 5/30, A61P 5/32, A61P 7/00, A61P 9/10, A61P 19/10, A61P 31/04, A61P 35/00, A61P 37/04, A61P 39/06, A61P 43/00, C07D 311/36

(54) **BIOLOGICAL ACTIVITY-PROMOTING COMPOSITIONS CONTAINING SOYBEAN GERM-ORIGIN ISOFLAVONE AGLYCON**

(30) Priority: 15.03.2002 JP 2002072939
(71) Applicant: Nichimo Co., Ltd, Tokyo 140-0002 (JP)
(72) Inventor: TAKEBE, Minoru, Nichimo Co., Ltd., Shinagawa-ku, Tokyo 140-0001 (JP)
(74) Representative: Ouzman, Beverley Nicola Claire
(86) International application number: PCT/JP2003/003157
(87) International publication number: WO 2003/077904

(57) **Abstract**

Biological activity-promoting compositions containing soybean germ-origin isoflavone aglycone which promote human or mammalian health and exert effective effects of preventing or treating diseases when absorbed into the body by oral administration, instillation, etc., thereby contributing to the maintenance and promotion of human or mammalian health, prevention of diseases and so on. The biological activity-promoting compositions are characterized by comprising an isoflavone aglycone concentrate which is prepared by fermenting the soybean germ with koji mold to thereby decompose proteins, then hydrolyzing the same and further extracting and concentrating the resultant product with a solvent. The compositions are further characterized in that the content of daidzein amounts is at least 70% by weight based on the isoflavone aglycone in the concentrate.

## Description

### Technological Field

The present invention relates to a biological activity-promoting composition that contains isoflavone aglycones derived from soybean germ, which stimulate health in humans and mammals and are effective in the prevention and treatment of diseases.

### Background Art

The applicant of the present application has previously disclosed, in Japanese Patent No. 3014145, that a large quantity of isoflavone aglycones is present in product obtained using beans (pulse crop) as raw material, enzymatically decomposing protein using koji mold, and then subjecting this material to hydrolysis and that this product acts to stimulate biological activity and has various pharmacological effects in humans and mammals.

The biological activity-promoting composition referred to in the present application refers to compositions that stimulate health in humans or mammals and are effective in the prevention or treatment of illnesses.

Examples of pharmacological effects are those that have been recently reported in regard to isoflavone compounds. For example, it is known that isoflavone compounds have biological activities such as estrogen action, antiestrogen action, antioxidant action, antihemolytic action, antimicrobial action, antilipidemic action, anticholesterol action, spasmolytic action, cancer cell differentiation induction action, oncogene inhibitory action, carcinostatic effect, arteriosclerosis inhibitory action and vertebrate immunostimulatory action. Among isoflavone compounds, isoflavone aglycones have high internal absorption rates, and there are many reports in regard to their pharmacological actions.

More specifically, genistein which is an aglycone that is produced from genistin by the hydrolysis of glycosidic glucose is known to be a substance that inhibits tyrosine kinase (TK blocker) that is necessary for the induction of carcinogenesis via oncogenes, and its carcinostatic effects have been confirmed (Akiyama et al., *Biochemistry* 59(9), 1016 (1987)).

In addition, genistein and daidzein have particularly strong estrogen actions among isoflavone compounds and have also been confirmed that they have osteoporosis therapeutic effects and immunostimulatory effects. With females in particular, osteoporosis is a particular problem after menopause, and one factor that may be cited is the decrease in bone mass that accompanies bone metabolic stimulation induced by estrogen deficiency.

However, the direct administration of estrogen which has been used as a treatment for osteoporosis is problematic in regard to cervical cancer, breast cancer and other side effects on reproductive organs. It would be thus effective from the standpoint of safety if it were possible to efficiently extract isoflavone compounds as effective components present in Leguminosae foods, particularly soybean, and to utilize them in the production of health foods or foods for mitigating symptomatic ailments.

Because isoflavone compounds have these pharmacological actions, their demand has intensified in recent years in the therapeutic and foodstuff industries. However, isoflavone compounds are present in small amounts in natural substances, and the amounts of isoflavone aglycones in particular are extremely small.

Isoflavone aglycones have been confirmed that they have superior pharmacological effects, among other properties, but a means for better manifesting these pharmacological effects is desired.

### Disclosure of Invention

The present invention was developed in light of these considerations, and its object is to provide a biological activity-promoting composition that contains isoflavone aglycones derived from soybean germ, which stimulates health in humans and mammals and has actions that are effective in the prevention or treatment of diseases.

The inventor of the present application, as a result of painstaking investigations, produced a product proposed previously in Japanese Patent No. 3014145, which is a "product that is obtained with the use of soybean germ as raw material bean (pulse crop), wherein koji preparation is performed on the soybean germ using koji mold, and its resultant is subjected to a hydrolysis treatment, thus obtaining a product containing a large amount of isoflavone aglycones or a product in which the phytic acid has been completely removed by decomposition"; and on this product, in accordance with the production method proposed in Japanese Patent Application Laid-Open (Kokai) No. 2000-281673 filed by the inventor of the present application and other inventor(s), with a use of solvent, extraction and concentration was performed, thus obtaining a product which is a concentrate with an isoflavone aglycone level of 30 wt% or greater (to which a mark "AglyMax" (Japanese registered trademark of Nichimo Co., Ltd., referred to below as "AglyMax") was applied). Upon comparing the administration of AglyMax and isoflavone aglycones alone to ovarectomized (OVX) rats, it was found that the pharmacological biological activity-promoting action in OVX rats administered AglyMax was dramatically superior relative to OVX rats having been administered isoflavone aglycones alone; thus realizing the invention of the present application.

The biological activity-promoting composition that contains isoflavone aglycones derived from soybean germ of the present invention obtained in this manner is characterized in that it is a product produced by fermenting soybean germ raw material with koji mold in order to break down the proteins, followed by hydrolysis thereon to obtain a resultant product that is then subjected to extraction and concentration using solvent, thus obtaining an isoflavone aglycone concentrate.

In addition, the present invention is characterized in that the composition of the isoflavone aglycones contained in the concentrate includes at least 70 wt% daidzein.

The biological activity-promoting composition that contains isoflavone aglycones derived from soybean germ of the present invention produced in this manner stimulates health in humans and mammals and manifests effective action with respect to the prevention or treatment of diseases. As a result, the composition can be used with favorable effect in the maintenance or promotion of health in humans and mammals and in the prevention of diseases.

### Brief Description of Drawings

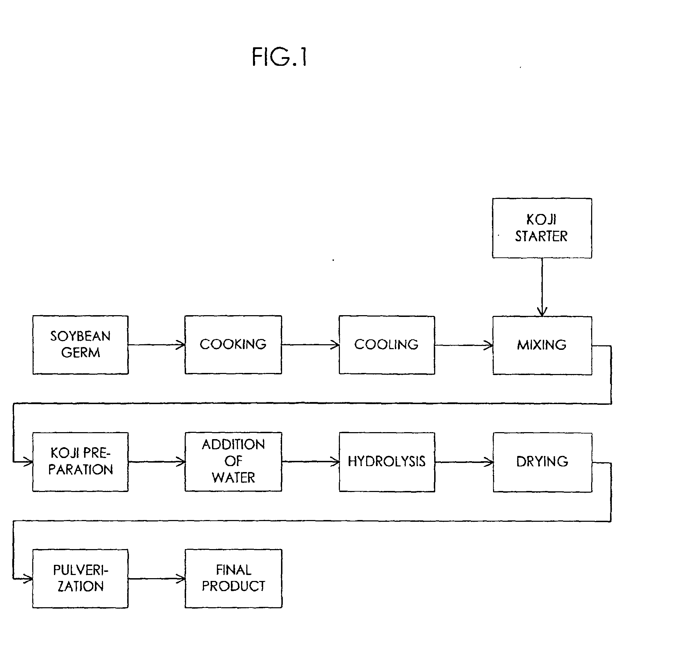

Figure 1 is a process diagram of one embodiment of the manufacturing method of the present invention that produces aglycones having favorable pharmacological effects from among the isoflavone compounds present in soybean germ.

### Best Mode for Carrying Out the Invention

Embodiments of the present invention will be described below.

In this embodiment, a product previously proposed by the inventor of the present application in Japanese Patent No. 3014145 is employed in which the "product is one obtained by using soybean germ as raw material bean (pulse crop), wherein koji preparation is performed on the soybean germ using koji mold, and its resultant is subjected to a hydrolysis treatment, so that the obtained product is a product which contains a large amount of isoflavone aglycones or is a product in which the phytic acid has been completely removed by decomposition"; and on the thus obtained product(s), in accordance with the production method disclosed in Japanese Patent Application Laid-Open (Kokai) No. 2000-28167 filed by the inventor of the present application and other inventor(s), solvent is used for extraction and concentration to produce a concentrate product containing isoflavone aglycones at, for example, 30 wt% or greater.

Figure 1 shows the product proposed in Japanese Patent No. 3014145 referred to above, and it is a process diagram that presents an embodiment of the product manufacture method wherein glycosides contained in the isoflavone compounds from the germ of soybean, which is a type of bean (pulse crop), are decomposed to generate isoflavone compounds rich in aglycones. Figure 1 also shows an embodiment for a method for manufacturing product produced by the removal of phytic acid in soybean meal.

First, a description will be presented regarding the manufacture of product that generates isoflavone compounds that are rich in aglycones.

In accordance with the process of Figure 1, soybean germ is first cooked, thereby facilitating the propagation of koji mold. The soybean germ can be cooked in a batch format or continuous format in accordance with factors such as manufacture objectives.

The soybean germ that has been cooked is then cooled, and the water content in the soybean germ is adjusted to an amount that allows the propagation of the koji mold (e.g., 37 wt%).

Using the soybean germ with the water content adjusted in this manner, the method of the present invention is then carried out in the manner described below.

More specifically, the cooked soybean germ is sterilized, and after cooling, the blending ratio of soybean germ and koji mold is set by mixing 400 kg of soybean germ and 200 g of koji starter with a koji mold count adjusted (using polished rice) to 8 x 10⁷/g. In addition, after cooling the mixture to 32°C at the start of koji preparation, the temperature of the product is allowed to increase to 40°C without providing ventilation, and temperature increase is then controlled by ventilating at the point when 40°C is reached. A first agitation is initiated at about 17 hours from the start ("*mori*" process). The soybean germ is cooled, and the temperature is controlled while ventilating so that the product temperature is about 35°C after agitation. Next, a second agitation process is performed after about eight hours ("*naka*" process), thus cooling the product. Again, the product temperature is controlled by ventilation at about 35°C; and after about 16 hours, a third agitation process is carried out in the same manner as above ("*shimai*" process). While subsequently ventilating so that the product temperature is controlled at about 38°C, koji preparation is completed at about 48 hours from the start. Upon completion of koji preparation, the water content is adjusted to 50% while agitation, and the product is heated so that the product temperature reaches about 50°C. Most of the isoflavone compounds in the soybean germ are converted to aglycones by the koji mold by hydrolysis for 48 hours or longer. As a result of the treatment carried out as shown in Table 1, a large quantity of isoflavone compounds of the soybean germ which contain daidzein aglycone as the primary component are obtained.

The koji mold used in the above koji preparation may be koji mold that is traditionally used in tempeh or traditional Japanese fermented foodstuffs. Examples include koji mold comprising *Aspergillus usami*, *Aspergillus kawachi, Aspergillus awamori*, *Aspergillus saitoi, Aspergillus oryzae, Aspergillus niger* and other *Aspergilli* or *Rhizopus* which are safe for use as foodstuffs.

The fermenting time depends on the type of koji mold that is used and is at least 24 hours, but a koji preparation time should be used that is sufficient to provide adequate decomposition of the isoflavone compound glycosides present in soybean meal.

Regarding protein hydrolysis, it is desirable for the hydrolysis temperature and hydrolysis time to be sufficient to allow adequate reduction in isoflavone compound glycoside amounts present in the soybean germ in accordance with the type of koji mold that is used.

By the means above, organic acid is generated during the initial period of koji preparation, and contaminant microorganism propagation in the soybean germ is inhibited, thereby eliminating worries concerning secondary contamination. As a result, mass production of product that uses soybean germ as a raw material is possible. In addition, a treatment can be carried out whereby the glycoside content in the isoflavone compounds is sufficiently reduced without reduced water content.

Next, in the present embodiment, in accordance with the method presented in Japanese Patent Application Laid-Open (Kokai) No. 2000-281673, with the use of the above resultant product obtained by hydrolysis (1) solvent extraction is performed using hydrophilic organic solvent, (2) liquid/liquid separation is performed using hydrophobic organic solvent on the hydrophilic organic solvent obtained by extraction, (3) liquid/liquid separation is performed using hydrophobic organic solvent on the hydrophilic organic solvent obtained by liquid/liquid separation, and (4) the hydrophobic organic solvent obtained by liquid/liquid extraction is subjected to concentration and drying as necessary. As a result, a concentrate having an isoflavone aglycone content of 30 wt% or greater as shown in Table 1 is thus obtained. The daidzein content is about 70 wt% of the isoflavone aglycones.

**Table 1**

| Analysis parameters | Standard values | Results |
|---|---|---|
| Condition | Light brown powder, no abnormal flavor, odor or impurities | Affirmative |
| Drying weight loss | 5% or less | 2.8% |
| Combustion residue | 3% or less | 0.1% |
| Arsenic | 2 ppm or less | Affirmative |
| Heavy metals | 20 ppm or less | Affirmative |
| Residual agrochemicals | Not detected | Not detected |
| Viable cell count | 3000/g or less | 3000/g or less |
| Coliforms | Negative | Negative |
| Isoflavone aglycone content | 30% or greater | 32.9% |
| Daidzein | | 23.4% |
| Glycitein | | 8.0% |
| Genistein | | 1.5% |

### Examples

A discussion will be presented below regarding the biological activity-promoting effects of the biological activity-promoting compositions of the present invention that contains isoflavone aglycones derived from soybean germ.

In this example, testing was carried out on females to facilitate measurement of the degree of stimulation of biological activity.

### 1) Female SD rats (seven weeks) were procured, and were preliminarily kept for one week. The healthy female SD rats to be used as subjects (eight weeks) were raised in groups with uniform body weight. Rats other than those in the Sham group were ovarectomized (OVX). The Sham group was only laparotomized. After preliminarily keeping for one week after the OVX procedure, the rats were allowed free intake of high-fat feed having the composition shown in Table 2 for a period of 28 days starting on the day following preliminary keeping.

The rats were also allowed free intake of well water (3 ppm chlorine added). In addition, the environment of the animal facility had constant temperature and humidity of 23 ± 2°C and 55 ± 10%. The atmosphere conditioning method involved ventilation (15 cycles/h), and the light time was from 8 am to 8 pm. The keeping apparatuses were stainless steel metabolic cages.

**Table 2**

| High-fat feed blending composition | wt% |
|---|---|
| Lard | 25.0 |
| Corn oil | 11.0 |
| Milk casein | 24.0 |
| Corn starch | 15.5 |
| Granular sugar | 10.0 |
| Avicel (crystalline cellulose) | 3.0 |
| KC flock (cellulose powder) | 2.0 |
| Okanol (pregelatinized starch) | 1.0 |
| Vitamin mix (CLEA, Inc. for purification/refinement [*lit*.]) | 1.0 |
| Mineral mix (CLEA, Inc. for purification/refinement [*lit*.]) | 7.0 |

### 2) Administration of feed

Six healthy female SD rats constituted the comparative group 1 (Sham group in the table), and six each of the OVX female SD rats were grouped in test group 1 (OVX group in the table), test group 2 (AglyMax group in the table), test group 3 (genistein group in the table), test group 4 (daidzein group in the table) and test group 5 (17 β-estradiol group in the table). In comparative group 1 and test group 2 only normal feed was given, whereas feed supplemented with AglyMax, genistein and daidzein was administered to test groups 2, 3 and 4 so that the amount of isoflavone aglycones was 20 mg per kg of body weight in each case. 17 β-estradiol was supplemented in the feed in test group 5 in the amount of 1 µg per animal per day.

### 3) Body weight measurement

The body weights of all rats were measured once on days 0, 7, 14, 21 and 28.

### 4) Feed intake measurement

The amount of feed taken in by all rats was measured once on days 0, 7, 14, 21 and 28.

### 5) Urological, hematological and other measurements

24-hour accumulated urine was collected during the administration of the high-fat feed, AglyMax, genistein, daidzein and 17 β-estradiol on days 0, 7, 14, 21 and 28, and urine NO₂/NO₃ measurements were performed. Upon completion of administration on day 28, 24-hour fasting was performed, and the rats were necropsied under ether anesthetization in order to collect blood from the abdominal aorta. Regarding serum cholesterol, measurement of total serum cholesterol, HDL cholesterol, LDL cholesterol and cholesterol ester ratio were carried out, and blood neutral fat (TG), blood free fatty acid (FFA) and leptin were measured using enzymatic methods. In addition, the uterus weight per 100 g body weight was measured for the necropsied females rats. Also, body fat determination was made by measuring the weight of removed posterior abdominal wall fat, mesenteric fat, perirenal fat and periovarian fat per 100 g of body weight. The thymus weights for the necropsied female rats were measured based on the weight per 100 g of body weight.

### 6) Statistical data processing

The results of measurement for each group were expressed as mean values ± standard deviations for each of the measured parameters, and significant differences were determined using Student's t-test. Evaluation was made using the two-sided test, where significance levels were taken as 5 and 1%.

### b) Test results

### 1) Observation of condition

No particular abnormalities were observed in regard to general observation of condition during the testing period.

### 2) The results of body weight measurement are shown in Table 3

**Table 3**

| Change in Body Weight (g) | | | | | |
|---|---|---|---|---|---|
| (Day) | | 0 | 7 | 14 | 21 |
| Sham group | (n=6) | 194.3±7.50 | 208.2±6.43 | 220.7±5.16 | 231.0±5.76 |
| OVX group | (n=6) | 195.7±5.92 | 214.3±8.27 | 233.7±8.84 | 254.4±8.02 |
| AglyMax group | (n=6) | 194.6±8.21 | 212.7±7.80 | 230.5±8.13 | 244.6±8.21 |
| genistein group | (n=6) | 197.3±10.18 | 216.9±8.53 | 231.9±9.77 | 249.4±9.81 |
| daidzein group | (n=6) | 191.0±7.31 | 216.1±6.86 | 230.6±6.58 | 245.4±7.07 |
| estradiol group | (n=6) | 192.5±7.12 | 206.5±8.58 | 218.6±10.20* | 226.9±10.77** |
| | | | | | |

| (Day) | | 28 | | | |
|---|---|---|---|---|---|
| Sham group | (n=6) | 241.0±6.32 | | | |
| OVX group | (n=6) | 271.5±6.28 | | | |
| AglyMax group | (n=b) | 254.1±7.97** | | | |
| genistein group | (n=6) | 266.8±8.6 | | | |
| daidzein group | (n=6) | 257.4±7.71** | | | |
| estradiol group | (n=6) | 235.5±9.24** | | | |
| (Mean ± standard deviation; Significance with respect to OVX group *: p<0.05; ** p<0.01) The estradiol group in the table denotes the 17β-estradiol group. | | | | | |

### 3) The results of measurement of feed intake are shown in Table 4

**Table 4**

| Change in Feed Intake Amount (g) | | | | | |
|---|---|---|---|---|---|
| (Day) | | 0 | 7 | 14 | 21 |
| Sham group | (n=6) | 16.6±0.56 | 17.1±0.48 | 17.6±0.38 | 17.7±0.72 |
| OVX group | (n=6) | 16.7±0.63 | 17.2±0.66 | 17.8±0.43 | 18.3±0.60 |
| AglyMax group | (n=6) | 16.5±0.59 | 17.0±0.59 | 17.5±0.50 | 18.1±0.66 |
| genistein group | (n=6) | 16.5±1.55 | 16.8±1.58 | 17.3±1.64 | 17.7±1.24 |
| daidzein group | (n=6) | 16.3±0.89 | 16.7±0.90 | 17.2±0.92 | 17.7±0.91 |
| estradiol group | (n=6) | 16.7±0.52 | 17.0±0.45 | 17.3±0.49 | 17.6±0.67 |
| | | | | | |

| (Day) | | 28 | | | |
|---|---|---|---|---|---|
| Sham group | (n=6) | 18.3±0.56 | | | |
| OVX group | (n=6) | 19.0±0.42 | | | |
| AglyMax group | (n=6) | 18.5±0.50 | | | |
| genistein group | (n=6) | 18.1±1.3 | | | |
| daidzein group | (n=6) | 16.3±0.85 | | | |
| estradiol group | (n=6) | 17.8±0.87 | | | |
| (Mean ± standard deviation; Significance with respect to OVX group *: p<0.05; ** p<0.01) The estradiol group in the table denotes the 17 β-estradiol group. | | | | | |

### 4) The results of urinary NO₂/NO₃ measurement are shown in Table 5

**Table 5**

| Change in NO₂/NO₃ (µmol) | | | | | |
|---|---|---|---|---|---|
| (Day) | | 0 | 7 | 14 | 21 |
| Sham group | (n=6) | 9.0±1.26** | 9.2±1.82* | 8.9±1.22** | 8.7±0.99** |
| OVX group | (n=6) | 6.7±0.89 | 6.6±0.82 | 7.0±0.75 | 6.8±0.84 |
| AglyMax group | (n=6) | 6.6±0.93 | 7.1±0.91 | 7.6±0.93 | 7.8±0.76* |
| genistein group | (n=6) | 6.1±0.34 | 6.4±0.39 | 6.3±0.56 | 6.2±0.40 |
| daidzein group | (n=6) | 6.0±0.53 | 6.4±0.54 | 6.4±0.43 | 6.6±0.40 |
| estradiol group | (n=6) | 6.1±0.29 | 7.0±0.60 | 7.8±0.59 | 9.0±0.76* |
| | | | | | |

| Day | | 28 | | | |
|---|---|---|---|---|---|
| Sham group | (n=6) | 9.2±1.62* | | | |
| OVX group | (n=6) | 6.9±0.83 | | | |
| AglyMax group | (n=6) | 8.3±0.83* | | | |
| genistein group | (n=6) | 6.4±0.4 | | | |
| daidzein group | (n=6) | 6.0±0.40 | | | |
| estradiol group | (n=6) | 9.4±0.39** | | | |
| (Mean ± standard deviation; Significance with respect to OVX group *: p<0.05; ** p<0.01) The estradiol group in the table denotes the 17 β-estradiol group. | | | | | |

### 5) The results of measuring serum cholesterol as total serum cholesterol, HDL cholesterol, LDL cholesterol and cholesterol ester ratio are shown in Table 6.

**Table 6**

| Change in Serum Cholesterol (mg/dl) | | | | | |
|---|---|---|---|---|---|
| (Day) | | Total cholesterol | HDL cholesterol | LDL cholesterol | Cholesterol ester (%) |
| Sham group | (n=6) | 72.8±5.00** | 37.2±2.59** | 35.4±1.59** | 70.9±1.46 |
| OVX group | (n=6) | 90.9±3.70 | 45.9±2.95 | 46.0±3.02 | 71.1±2.14 |
| AglyMax group | (n=6) | 79.1±2.55** | 44.4±1.10 | 32.6±1.84** | 72.6±1.27 |
| genistein group | (n=6) | 85.8±2.53* | 47.8±1.48 | 40.6±1.76** | 70.5±1.48 |
| daidzein group | (n=6) | 81.8±2.57** | 42.6±2.25 | 36.3±3.45** | 70.1±3.01 |
| estradiol group | (n=6) | 72.1±3.86** | 35.9±3.38** | 38.2±1.74** | 70.7±2.80 |
| (Mean ± standard deviation; Significance with respect to OVX group *: p<0.05; ** p<0.01) The estradiol group in the table denotes the 17 β-estradiol group. | | | | | |

### 6) The results of measuring blood neutral fat (TG) are shown in Table 7

**Table 7**

| Change in TG (mg/dl) | | |
|---|---|---|
| (Day) | | TG |
| Sham group | (n=6) | 107.2±11.25** |
| OVX group | (n=6) | 130.5±12.34 |
| AglyMax group | (n=6) | 109.9±12.25* |
| genistein group | (n=6) | 116.9±9.90 |
| daidzein group | (n=6) | 104.8±16.71* |
| estradiol group | (n=6) | 105.1±5.37** |
| (Mean ± standard deviation; Significance with respect to OVX group *: p<0.05; ** p<0.01) The estradiol group in the table denotes the 17 β-estradiol group. | | |

### 7) The results of measuring blood free fatty acid (FFA) are shown in Table 8

**Table 8**

| Change in FFA (mg/dl) | | |
|---|---|---|
| (Day) | | TG |
| Sham group | (n=6) | 347.5±26.94** |
| OVX group | (n=6) | 404.5±17.57 |
| AglyMax group | (n=6) | 349.0±25.68** |
| genistein group | (n=6) | 400.0±19.01 |
| daidzein group | (n=6) | 379.2±24.45 |
| estradiol group | (n=6) | 362.8±24.41** |
| (Mean ± standard deviation; Significance with respect to OVX group *: p<0.05; ** p<0.01) The estradiol group in the table denotes the 17 β-estradiol group. | | |

### 8) The results of measuring leptin levels are shown in Table 9

**Table 9**

| Change in Leptin (ng/ml) | | |
|---|---|---|
| (Day) | | Leptin |
| Sham group | (n=6) | 1.0±0.15** |
| OVX group | (n=6) | 2.1±0.23 |
| AglyMax group | (n=6) | 1.3±0.22** |
| genistein group | (n=6) | 1.9±0.25 |
| daidzein group | (n=6) | 1.7±0.12** |
| estradiol group | (n=6) | 0.7±0.28** |
| (Mean ± standard deviation; Significance with respect to OVX group *: p<0.05; ** p<0.01) The estradiol group in the table denotes the 17 β-estradiol group. | | |

### 9) The results of measuring uterus weight are shown in Table 10.

**Table 10**

| Change in Uterus Weight (mg/100g) | | |
|---|---|---|
| (Day) | | Uterus weight |
| Sham group | (n=6) | 1.7±0.07** |
| OVX group | (n=6) | 0.9±0.06 |
| AglyMax group | (n=6) | 1.4±0.09** |
| genistein group | (n=6) | 0.9±0.06 |
| daidzein group | (n=6) | 1.3±0.08** |
| estradiol group | (n=6) | 1.6±0.08** |
| (Mean ± standard deviation; Significance with respect to OVX group *: p<0.05; ** p<0.01) The estradiol group in the table denotes the 17 β-estradiol group. | | |

### 10) The results of measuring posterior abdominal wall fat, mesenteric fat, perirenal fat and periovarian fat are shown in Table 11.

**Table 11**

| Change in Body Fat (g/100g; body weight) | | | | | |
|---|---|---|---|---|---|
| (Day) | | posterior abdominal wall fat | Mesenteric fat | Perirenal fat | Periovarian fat |
| Sham group | (n=6) | 2.0±0.12** | 2.3±0.28** | 3.0±0.28** | 2.6±0.23** |
| OVX group | (n=6) | 2.9±0.23 | 2.9±0.27 | 4.0±0.19 | 3.1±0.17 |
| AglyMax group | (n=6) | 2.3±0.23** | 2.6±0.24* | 3.3±0.14** | 2.9±0.17 |
| genistein group | (n=6) | 2.8±0.25 | 2.6±0.14 | 3.7±0.17* | 3.0±0.26 |
| daidzein group | (n=6) | 2.6±0.23 | 2.8±0.18 | 3.5±0.20** | 3.1±0.17 |
| estradiol group | (n=6) | 1.9±0.10** | 2.5±0.19* | 3.2±0.22** | 2.8±0.27* |
| (Mean ± standard deviation; Significance with respect to OVX group *: p<0.05; ** p<0.01) The estradiol group in the table denotes the 17 β-estradiol group. | | | | | |

### 11) The results of measuring thymus weight are shown in Table 12

**Table 12**

| Change in Thymus Weight (g/100g; body weight) | | |
|---|---|---|
| | (Day) | Thymus weight |
| Sham group | (n=6) | 0.118±0.02 |
| OVX group | (n=6) | 0.119±0.01 |
| AglyMax group | (n=6) | 0.129±0.01** |
| genistein group | (n=6) | 0.110±0.02** |
| daidzein group | (n=6) | 0.130±0.02** |
| estradiol group | (n=6) | 0.112±0.01** |
| (Mean ± standard deviation; Significance with respect to OVX group *: p<0.05; ** p<0.01) The estradiol group in the table denotes the 17 β-estradiol group. | | |

### c) Evaluation

For all measured parameters, the measurement results for test group 2 in which the AglyMax of the present invention was used were found to be dramatically superior in terms of biological activity promotion relative to test group 3 and test group 4 that received only isoflavone aglycones. In addition, because the 17 β-estradiol administered to test group 5 is an estrogen, its stimulatory (agonistic) action with respect to biological activity was high, even at an administered amount of 1/4000 relative to isoflavone aglycones. Consequently, results were obtained that caused concern over side effects when administered at high dosages. More specifically, the estrogen 17 β-estradiol may cause extremely strong side effects, and is a substance that should only be administered in a reduced amount of about 1/4000.

Presumably, the reason that AglyMax of the present invention has superior action relative to isoflavone aglycones is because the AglyMax is produced using natural soybean germ as raw material, and because the isoflavone aglycones, which are produced by koji preparation, by decomposition of the soybean germ under the decomposition action of koji mold enzymes and by hydrolysis, are natural, and further because, when considering the component ratios, its daidzein content is 70 wt% which is far greater than the other genistein and glycitein aglycones.

The thymus is an organ that has an important immunological function, specifically in relation to T-cell differentiation, and thymus weight decreased in the genistein test group 3 and the 17 β-estradiol test group 5 relative to the comparative group 1 and the test group 1. However, it was found that the AglyMax test group 2 provided superior performance in that an increase in weight was seen that was similar to that of the daidzein test group 4. In addition, effective stimulation of T-cell differentiation by the biological activity-promoting composition that contains isoflavone aglycones derived from soybean germ of the present invention can be confirmed by microscopic observation of thymus tissue specimens.

Moreover, the superior effects described above can be manifested by reliable absorption through the digestive organs when the biological activity-promoting composition of the present invention having weak estrogen-like action containing isoflavone aglycones derived from soybean germ is formed into a capsule, tablet, granule, powder or other form, thus facilitating oral administration.

The present invention can be modified as necessary and is not restricted by the embodiments and examples presented above. The biological activity-promoting composition that contains isoflavone aglycones derived from soybean germ of the present invention can be absorbed into the body by means of instillation as well as oral administration.

## Claims

1. A biological activity-promoting composition that contains isoflavone aglycones derived from soybean germ, **characterized in that** said composition is obtained by fermenting soybean germ, which is used as raw material, with koji mold, thus braking down proteins, then executing hydrolysis thereon, and a resultant thereof is extracted and concentrated using solvent to produce an isoflavone aglycone concentrate.

2. The biological activity-promoting composition that contains isoflavone aglycones derived from soybean germ according to claim 1, wherein the composition of the concentrated isoflavone aglycones includes at least 70 wt% daidzein.
